# EUROPEAN PATENT APPLICATION

(11) **EP 4 523 602 A1**
(43) Date of publication of application: **19.03.2025**
(21) Application number: 24200025.5
(22) Date of filing: 12.09.2024
(51) Int. Cl.: A61B 1/12, A61B 1/00, A61B 90/70

(54) **METHOD AND ARRANGEMENT FOR PRE-CLEANING AN ENDOSCOPE**

(30) Priority: 13.09.2023 US 202363538114 P
(71) Applicant: Olympus Winter & Ibe GmbH, 22045 Hamburg (DE)
(72) Inventor: Ottens, Benjamin, 22399 Hamburg (DE)
(74) Representative: Eisenführ Speiser

(57) **Abstract**

The invention relates to a method for pre-cleaning an endoscope. Further, the invention relates to an arrangement for pre-cleaning an endoscope. It is provided a method for pre-cleaning an endoscope, preferably immediately or shortly after use of the endoscope, the method comprising: providing an endoscope that comprises channels for transporting fluids, connection ports, in particular for connection of fluid hoses, and a distal end of the endoscope, wherein each of the channels extends from one of the connection ports to the distal end of the endoscope; fluidly connecting the channels to each other at the distal end of the endoscope; connecting a suction device, preferably a suction pump, to at least one of the connection ports or to the distal end of the endoscope; connecting at least one vessel that holds liquid to at least one of the connection ports; flushing at least one of the channels with liquid by activating the suction device so that liquid is drawn from the at least one vessel through the at least one channel towards the suction device.

## Description

### TECHNICAL FIELD

The invention relates to a method for pre-cleaning an endoscope. Further, the invention relates to an arrangement for pre-cleaning an endoscope.

### BACKGROUND

An endoscope is an inspection instrument that can be used to examine, and optionally manipulate, the inside of organisms. Endoscopes can be used in human medical diagnostics for examinations or minimally invasive surgical procedures on humans or animals. Endoscopes typically comprise an image sensor, an optical lens, and a light source.

After use of an endoscope, the endoscope is typically pre-cleaned, manually or automated cleaned, disinfected, and possibly sterilized before being used again on a patient. If an endoscope and accessories used in the patient procedure are not immediately cleaned after each patient procedure, residual organic debris will begin to dry and solidify, hindering effective removal and reprocessing efficacy. Therefore, it is necessary to pre-clean the endoscope and the accessories at the bedside immediately after each patient procedure.

Typically, the pre-cleaning that is conducted at the bedside immediately after each patient procedure inter alia comprises the following steps: First, a biopsy/suction channel is pre-cleaned by arranging a distal end of the endoscope in water, and drawing the fluid through the endoscope. Then, air is drawn through the endoscope. Second, an air/water channel is pre-cleaned by arranging the distal end of the endoscope in water and flush the air/water channel with the fluid. Then, the air/water channel is flushed with air. Third, if available, an auxiliary water channel is pre-cleaned by flushing the auxiliary water channel with water. Finally, the accessories are detached from the endoscope and placed in a fluid, such as water or a detergent solution. Typically, after the pre-cleaning procedure the endoscope is detached from the equipment (such as for example video system center, light source, suction pump) used in the patient procedure and transported to a reprocessing area.

In US 11445900B2 it was suggested to pre-clean an endoscope by using a plurality of input members in form of flexible tubes for use in delivering cleaning solution to multiple input ports of an endoscope, so that a cleaning solution is delivered through the flexible tubes to the endoscope.

However, known procedures for pre-cleaning are both relatively time-consuming and prone to errors. As the pre-cleaning typically consists of several subsequently conducted manually applied steps, the pre-cleaning is relatively time-consuming and if only one of these steps is not conducted exactly according to the pre-cleaning procedure, the endoscope might not be pre-cleaned sufficiently. Furthermore, because not all endoscope channels are connected to each other it is not possible to flush all channels with only one source. In typically conducted pre-cleaning procedures, the video system center is required to flush the air/water channel, a suction pump to aspire the suction and biopsy channel and a flushing pump or syringe to flush the auxiliary water channel. Therefore, the user is permanently involved in the procedure and needs to interact with several different user interfaces which requires that the user can operate all of these user interfaces. Additionally a lot of equipment/accessories is necessary to conduct the pre-cleaning procedure.

Furthermore, a problem is that after pre-cleaning, drying and solidification can occur in the channels that are no longer filled with fluid.

Furthermore, the fluid flow and fluid volume used for flushing the channels of the endoscope is typically controlled manually, i.e. by pushing valves, by the user who performs the pre-cleaning procedure. Thus, the contact time of fluid in the channels is difficult to control, as it is dependent on the manual control of the user. The contact time of the fluid in the channels is limited to the corresponding pre-cleaning step itself as the fluid is removed from the channels after the pre-cleaning procedure. There is a risk that remaining soil can dry after conducting the pre-cleaning procedure.

Furthermore, blockages of channels can occur that can lead to insufficient pre-cleaning of these channels, because fluid cannot pass such blockages so that channels or parts of channels stay dry and are not in contact with the fluid and thus not pre-cleaned sufficiently.

### SUMMARY

Therefore, it is an object of the present invention to provide an improved method and an improved arrangement for pre-cleaning an endoscope. In particular, it is an object of the present invention to provide a method for pre-cleaning an endoscope that is less prone to errors.

According to a first aspect, it is provided a method for pre-cleaning an endoscope, preferably immediately or shortly after use of the endoscope, the method comprising: providing an endoscope that comprises channels for transporting fluids, connection ports, in particular for connection of fluid hoses, and a distal end of the endoscope, wherein each of the channels extends from one of the connection ports to the distal end of the endoscope; fluidly connecting the channels to each other at the distal end of the endoscope; connecting a suction device, preferably a suction pump, to at least one of the connection ports or to the distal end of the endoscope; connecting at least one vessel that holds liquid to at least one of the connection ports; flushing at least one of the channels with liquid by activating the suction device so that liquid is drawn from the at least one vessel through the at least one channel towards the suction device.

In the following, initially the method for pre-cleaning an endoscope and its functions and advantageous embodiments will be described.

The distal end of the endoscope may also be referred to as the distal tip of the endoscope. Preferably, the distal end is to be understood as the end portion of the endoscope that is located at the very front of an insert section of the endoscope, in particular the end portion that is first inserted into the patient when the insert section of the endoscope is moved into a patient. Preferably, outlets of the channels are arranged at the distal end. Further preferably, a light guide is arranged at the distal end. Further preferably, an objective lens can be arranged at the distal end. It is particularly preferred if the distal end is arranged at the end of an insert section that has a flexible insertion tube.

Preferably, the method is conducted immediately or shortly after use of the endoscope in a patient procedure. Preferably the method for pre-cleaning the endoscope is started within 60 minutes after being used in the patient procedure, particularly preferably within 30 minutes after being used in the patient procedure, in particular within 15 minutes after being used in the patient procedure. Preferably, the pre-cleaning is conducted at a patient bedside so that the method can be conducted immediately or shortly after use of the endoscope in a patient procedure without the need to move the endoscope to another location prior to conducting the pre-cleaning.

Preferably, at least two of the channels are flushed with a liquid by activating the suction device so that the liquid is drawn from the vessel that holds liquid through the channels to the suction device.

Preferably, the channels are fluidly connected to each other via arranging a container onto the distal end of the endoscope. Then the liquid can be drawn from the at least one vessel through a channel to the distal end and via the fluid connection provided by the container arranged at the distal end further through another channel to which the suction device is connected to, so that the liquid then flows from the distal end towards the suction device. If a container is arranged onto the distal end, the container can be arranged onto the distal end in a condition in which it is filled with liquid or in which it is not filled with liquid. If the container is not filled with liquid, a continuous flow of liquid can be drawn through the channels via the distal end. Preferably, before flushing channels of the endoscope, the container is not filled with liquid. An advantage of such an empty container is that liquid can be drawn from a connection port through a channel to the container and from the container through a suction channel towards the suction device out of the endoscope, wherein no gaps of liquid that contain gas are present. Thus, when liquid is drawn out of a connection port, then the user can be sure that this liquid has been drawn completely through at least two channels. However, alternatively, it is also possible that the container is already filled with liquid before flushing the channels. Filling the container with liquid before starting the flushing of the channels can have the advantage that the distal end of the endoscope is in contact with liquid for a longer period of time and soaked for a longer period of time, which can be beneficial to achieve a more thorough pre-cleaning of the distal end, if needed.

However, as an alternative to arranging such a container onto the distal end of the endoscope it is also possible to connect the suction device, preferably indirectly via a suction hose or the like, to the distal end of the endoscope. In this alternative way, also a fluid connection of the channels that extend to the distal end can be obtained at the distal end of the endoscope.

Preferably, the channels each extend from one of the connection ports to the distal end of the endoscope. However, the endoscope can also have additional channels that do not extend from one of the connection ports to the distal end.

Preferably, the connection ports are to be understood as ports to which fluid hoses or the like can be connected to.

Preferably, the endoscope can for example be a colonoscope, or a gastroscopes, or an ultrasonic endoscope, or a duodenoscopes, or another type of endoscope.

An advantage of this method is that even though not all channels of the endoscope are connected to each other inside of the endoscope, it is possible to flush all of these channels with only one source by connecting the cannels to each other. Therefore, the pre-cleaning procedure can be conducted faster, easier and in a way that is less prone to errors.

An advantage of this method is that the channels can be kept humid and/or filled with liquid after having conducted the pre-cleaning procedure. Thus, drying and solidification in the channels after pre-cleaning can be prevented in a particularly advantageous way.

Another advantage of this method is that during conducting the pre-cleaning of the endoscope it is not necessary to interact with several user interfaces and/or devices in order to conduct the pre-cleaning procedure.

Another advantage of this method is that compared to conventional pre-cleaning methods, with the described method, much less equipment and/or accessories is necessary to conduct the pre-cleaning. For example, no video center or flushing pump is necessary to conduct the pre-cleaning.

In a particularly preferred embodiment, the at least one vessel is fluidly connected to at least one of the connection ports in such a manner that the at least one vessel is sealed liquid-tight from the environment that surrounds the vessel.

Such a liquid-tight sealing of the at least one vessel is in particular to be understood as a sealing that prevents liquid to flow between the interior of the vessel and the outside of the vessel if the vessel is connected to a connection port. Preferably, then, the only fluid connection to the interior of the vessel is to the channel extending to the connection port to which the vessel is connected.

In a further preferred embodiment, the at least one vessel is fluidly connected to at least one of the connection ports in such a manner that the at least one vessel is sealed gas-tight from the environment that surrounds the vessel.

Such a gas-tight sealing of the at least one vessel is in particular to be understood as a sealing that prevents gas, in particular air, to flow between the interior of the vessel and the outside of the vessel if the vessel is connected to a connection port. Preferably, then, the only gas connection to the interior of the vessel is to the channel extending to the connection port to which the vessel is connected to.

An advantage of such a liquid-tight and gas-tight sealing is that with such a sealing it is possible to provide a vessel that can change its interior volume depending on the amount of liquid being drawn out of the vessel.

In a further preferred embodiment, the at least one vessel comprises a vessel holding chamber with a vessel holding chamber volume for holding the liquid, wherein the vessel holding chamber volume reduces, preferably by the volume of liquid that is drawn from the vessel, during the drawing of liquid from the vessel to the at least one channel.

Such a vessel holding chamber is in particular to be understood as the part of the vessel in which the liquid that is hold by the vessel is located. Preferably, the liquid is drawn from the vessel holding chamber via the connection port to which the vessel is connected to the at least one channel that extends to this connection port.

In a further preferred embodiment, the vessel holding chamber is completely filled with liquid at any time before all of the liquid has been drawn out of the vessel holding chamber.

Preferably, the vessel holding chamber is completely filled with liquid at any time before all of the liquid has been drawn out of the vessel holding chamber even if the volume of the vessel holding chamber changes as liquid is drawn out of the vessel. Then, preferably, the volume of the liquid in the vessel holding chamber as well as the volume of the vessel holding chamber are reduced, but the vessel holding chamber is still completely filled with liquid, because preferably the volume of the liquid and the volume of the vessel holding chamber reduce in the same way.

Therefore, preferably, the vessel is designed in such a way that the volume of the vessel holding chamber reduces if an underpressure is applied to draw liquid out of the vessel.

An advantage of such a changing volume of the vessel holding chamber is that it can be seen from the outside of the vessel how the volume is reduced over time and when all of the liquid has been drawn out of the vessel.

In a further preferred embodiment, the liquid is drawn out of the vessel holding chamber until the vessel holding chamber volume reaches its minimal volume so that remaining liquid in the at least one channel stops to flow towards the suction device.

An advantage of such a vessel with such a vessel holding chamber that reduces its volume when liquid is drawn out of the vessel is that if the vessel is empty and the minimum volume of the vessel holding chamber is reached, the flow through the channel to which the vessel is connected automatically stops because preferably, in this state, the underpressure is not strong enough to draw the remaining liquid that is only located in the channel completely out of the channel.

A further advantage is that due to a stable state of the liquid in the channels after having removed the endoscope from the suction device, it can be decided actively when the channels are emptied.

In a further preferred embodiment, the vessel holding chamber is formed by a plastic bag, and/or wherein the vessel comprises a height-adjustable structure that reduces its height while liquid is drawn out of the vessel holding chamber.

Preferably, the vessel holding chamber is made of an elastic material and/or an easily deformable material, so that when liquid is drawn out of the vessel, the vessel holding chamber deforms in such a way that the volume of the vessel holding chamber is reduced by the amount of volume that is drawn out of the vessel holding chamber.

In a further preferred embodiment, the method comprises: when the liquid is drawn out of the vessel holding chamber, disconnecting the connection between the suction device and the at least one connection port to which the suction device had been connected.

An advantage is that when the underpressure is removed, which is in particular the case if the endoscope is disconnected from the suction device, the liquid stays in the channels due to capillary action in the channels. In this way, the wet status of the endoscope channels can be maintained for a long time, i.e. as long as necessary. Such a wet status is particularly beneficial because a drying of potential soiling can be avoided and, in case of a detergent solution (in particular in case of enzymatic detergents) used as liquid, additionally the contact time and/or immersion time can be extended to improve cleaning efficacy.

It is particularly preferred to connect each of the connection ports either to a vessel or to a closure element or to the suction device. Preferably, then each of the connection ports is connected to a vessel or to a closure element or, preferably indirectly via a hose or the like, to the suction device.

Closure elements are preferably to be understood as elements that are adapted to close a connection port, preferably fluid-tight, in particular air-tight, when being applied onto the connection port. Preferably, closure elements are connected to such connection ports that are connected to a channel that does not need to be flushed in the pre-cleaning procedure.

In a further preferred embodiment, a hydrophobic filter is arranged at each of the at least one vessel, wherein the hydrophobic filter is designed to allow gas to flow from the environment that surrounds the vessel via the hydrophobic filter into the vessel holding chamber of the corresponding vessel.

Preferably, the hydrophobic filter is designed to prevent liquid to flow from the environment that surrounds the vessel via the hydrophobic filter into the vessel holding chamber of the corresponding vessel.

Preferably, the hydrophobic filter is designed to prevent liquid to flow from the vessel holding chamber of the vessel via the hydrophobic filter to the environment that surrounds the corresponding vessel but to allow gas, in particular air, to enter the vessel holding chamber from the environment that surrounds the corresponding vessel.

Preferably the hydrophobic filter is arranged opposite to the connection area of the vessel that is connected to the connection port.

An advantage of an arrangement with such hydrophobic filters at the vessels it is possible to draw liquid from the vessels through the channels and after the liquid has been drawn out of the vessels to automatically draw air through the channels, if the underpressure applied to draw the liquid through the channels is upheld.

In a further preferred embodiment, the method comprises: after flushing at least one of the channels with liquid, flushing at least one of the channels with air by keeping the suction device activated after the liquid has been drawn out from the at least one vessel to draw air from the environment that surrounds the vessel via the hydrophobic filter into the vessel holding chamber of the corresponding vessel and further through the at least one channel towards the suction device.

An advantage of such a method is that liquid is drawn from the vessels through the channels directly followed by air. Then the air flow can be stopped by switching of the suction device. Advantageously, the user conducting the pre-cleaning procedure does not need to actively control the liquid flow, because precisely the amount of liquid that is arranged in the vessel holding chamber is drawn through the corresponding channels. The user does not need to actively stop the liquid flow. The user can then turn off the suction device and/or disconnect the suction device from the endoscope to stop the air flow.

In a further preferred embodiment, fluidly connecting the channels to each other at the distal end of the endoscope is conducted by arranging a container onto the distal end of the endoscope.

Preferably, liquid is drawn from the vessels into the channels via the connection ports of the channels, wherein the liquid is drawn through these channels towards the distal end and then via the fluid connection provided by the container at the distal end through the at least one channel that is connected to the suction device, i.e. the suction channel. After all the liquid is drawn out of these vessels, due to the presence of the hydrophobic filters, air is drawn in the same way through the channels via the distal end and towards the suction device until the underpressure is turned off, for example by switching off the suction device.

In a further preferred embodiment, the container is arranged on the distal end of the endoscope in such a manner that the distal end of the endoscope is sealed liquid-tight, and preferably gas-tight, from the environment that surrounds the container.

An advantage of sealing the inside of the container against the environment is that in this way no external fluids can enter the channels and no liquid can flow out from the container in the environment that surrounds the container.

Preferably, the inner volume of the container is not changeable. This can be achieved, for example, by using a relatively stiff material for the wall of the container that defines an inner chamber of the container. This way, when an underpressure is applied to the inner chamber of the container, the inner volume of the container does not contain but remains substantially unchanged so that the fluid connection of the channels at the end of the endoscope remains even when an underpressure is applied to the channel system and thus to the inner volume of the container.

In a further preferred embodiment, when flushing at least one of the channels with a liquid, liquid is drawn from the at least one vessel through the at least one channel via the distal end towards the suction device.

Preferably, at least two of the channels are flushed with a liquid by activating the suction device so that the liquid is drawn from the vessel that holds liquid through the channels to the suction device.

In a further preferred embodiment, the channels comprise an air channel that extends from an air channel connection port to the distal end of the endoscope, a suction channel that extends from a suction channel connection port to the distal end of the endoscope, a water channel that extends from a water channel connection port to the distal end of the endoscope, and preferably an auxiliary water channel that extends from an auxiliary water channel connection port to the distal end of the endoscope.

Preferably, the air channel is adapted to provide air to the distal end of the endoscope. Preferably, the suction channel is adapted to draw liquids and/or objects, such as for example tissue samples, from the distal end of the endoscope through the endoscope. Preferably, the water channel is adapted to provide water at the distal end of the endoscope. Preferably, the auxiliary water channel is adapted to provide water at the distal end of the endoscope.

Preferably, the channels are arranged parallel to each other over at least a part of their extension from one of the connection ports to the distal end of the endoscope.

In a further preferred embodiment, the method comprises: disconnecting a fluid connection between the air channel and the water channel, preferably by arranging a sealing element at an air/water cylinder, for separating the air channel from the water channel; and preferably drawing liquid from the at least one vessel through at least one of the channels towards the suction device.

Preferably the air channel and the water channel can be fluidly connected to each other, wherein this fluid connection is disconnected to allow for independent liquid flow in each of these channels. Preferably disconnecting such a fluid connection between the air channel and the water channel is conducted by applying a sealing element at a position where these channels can be connect. Preferably, the sealing is arranged within an air/water cylinder in which the channels are connectable to each other to disconnect the channels from each other by the sealing. Then, vessels can be connected to the connection ports of the water channel and of the air channel and a suction device can be connected for example to a connection port of a suction channel or to the distal end of the endoscope to draw liquid from the vessels through the air channel and through the water channel, wherein the liquid is drawn through these disconnected channels independently.

Preferably in addition to disconnecting such a fluid connection, each of the connection ports is connected either to a vessel or to a closure element or to the suction device. Preferably, then each of the connection ports is connected to a vessel or to a closure element or, preferably indirectly via a hose or the like, to the suction device. This way, advantageously, all channels to which a vessel is connected, can be flushed with liquid independently.

Preferably, after a defined runtime of the suction device, the vessels are emptied, i.e. the liquid is drawn out of the vessels through the channels, if no blockage is present in the channels that prevents liquid flow through the channels. Preferably, the user can verify if the vessels are empty, i.e. if the liquid has been drawn out of each of the vessels. This way, in a particularly advantageous way, it is possible to check if a channel is blocked and in case at least one of the channels is blocked, which channel or which channels are blocked.

Preferably, having applied an underpressure to draw liquid through the channels, based on the filling levels of the vessels it can be identified if at least one of the channels is blocked and which of the channels is blocked. If a vessel remains full the channel to which the vessel is connected via the connection port of this channel is blocked.

In an example, a first vessel can be connected to an air feed connection port of a first branch of the air channel, a second vessel can be connected to a water bottle connection port of the second branch of the air channel, wherein the first branch and the second branch of the air channel are fluidly connected at a connection point to then form one air channel that extends to the distal end, and a third vessel can be connected to a water bottle connection port of the water channel. In such an example, it is possible to check if a channel is blocked and in case at least one of the channels is blocked, which channel or which channels are blocked. This check can be done by analyzing the filling levels of liquid in the vessels as follows: If, after having applied an underpressure to flush the channels, liquid remains in the first vessel and no liquid is left in the second vessel and no liquid is left in the third vessel, the first branch of the air channel to which the first vessel is connected is blocked. If, after having applied an underpressure to flush the channels, liquid remains in the second vessel and no liquid is left in the first vessel and no liquid is left in the third vessel, the second branch of the air channel to which the second vessel is connected is blocked. If, after having applied an underpressure to flush the channels, liquid remains in the first vessel and liquid remains in the second vessel and no liquid is left in the third vessel, either the first branch and the second branch of the air channel or the air channel is blocked. If, after having applied an underpressure to flush the channels, liquid remains in the third vessel and no liquid is left in the first vessel and no liquid is left in the second vessel, the water channel to which the third vessel is connected is blocked. If, after having applied an underpressure to flush the channels, liquid remains in the first vessel and liquid remains in the second vessel and liquid remains in the third vessel, the fluid connection that is provided at the distal end, preferably by a container arranged onto the distal end, is blocked.

It is particularly preferred that the vessels are transparent. Providing transparent vessels has the advantage, that it can be seen by a user from outside of the vessels how much liquid is left in the vessels.

In a further preferred embodiment, the method comprises: checking, preferably after a predetermined time of activation of the suction device, whether liquid is left in each of the vessels, wherein preferably each of the vessels contains a code that is readable if no liquid is left in the vessel, and preferably not readable if liquid is left in the vessel, wherein preferably the patency of the channel that is connected to the vessel is determined and/or proofed by reading out the code.

Preferably, the code can only be read out if the corresponding vessel is empty, i.e. if the liquid has completely been drawn out of this vessel. Preferably, the code cannot be read out if the corresponding vessel is not empty, i.e. if the liquid has not completely been drawn out of this vessel.

An advantage of providing such a code is that with such a code it is possible to verify and document in a reliable way whether the corresponding vessel is empty and thus whether the channel to which the vessel is connected has been flushed with the liquid that was in the vessel. Thus, such a codes provides for a reliable documentation on which channels have been flushed, wherein the documentation cannot be manipulated, because the code can only be read out if the vessel is empty and thus if the corresponding channel has been flushed with the liquid. This way, such codes can in particular prevent errors in documentation and therefore increase the reliability that all channels have been cleaned.

In a further preferred embodiment, the method comprises: assessing, preferably after a predetermined time of activation of the suction device, whether liquid is left in each of the vessels, and dependent on the result of the assessment, determining whether at least one of the channels is blocked, and preferably if at least one of the channels is blocked, determining which of the channels is blocked.

A further advantage of providing such a code is that with such a code it is possible to verify and document in a reliable way the patency of the channels.

In a further preferred embodiment, fluidly connecting the channels to each other at the distal end of the endoscope is conducted by arranging a container onto the distal end of the endoscope, wherein the container is arranged on the distal end of the endoscope in such a manner that the distal end of the endoscope is sealed liquid-tight, and preferably gas-tight, from the environment that surrounds the container, wherein preferably each of the connection ports is connected to one of the at least one vessel except for the connection port that is connected to the suction device.

The advantages explained above with respect to the method for pre-cleaning are also advantages for such an arrangement for pre-cleaning.

According to a further aspect, it is provided an arrangement for pre-cleaning an endoscope, preferably immediately or shortly after use of the endoscope, in particular for pre-cleaning an endoscope according to the method as described herein, the arrangement comprising: an endoscope that comprises channels for transporting fluids, connection ports, in particular for connection of fluid hoses, and a distal end of the endoscope, wherein each of the channels extends from one of the connection ports to the distal end of the endoscope; at least one vessel that holds liquid, wherein the at least one vessel is connected to at least one of the connection ports; a suction device, preferably a suction pump, that is connected to one of the connection ports or to the distal end of the endoscope.

In a preferred embodiment, the arrangement comprises: a container that is arranged onto the distal end of the endoscope in such a manner that the channels are fluidly connected to each other at the distal end of the endoscope, wherein the container is arranged on the distal end of the endoscope in such a manner that the distal end of the endoscope is sealed liquid-tight, and preferably gas-tight, from the environment that surrounds the container, wherein preferably each of the connection ports is connected to one of the at least one vessel except for the connection port that is connected to the suction device.

As to the advantages, preferred embodiments and details of the individual different aspects and their preferred embodiments, reference is also made to the corresponding advantages, preferred embodiments and details described with reference to the respective other aspects.

Further advantageous embodiments result from the combination of individual, several or all of the preferred features described herein.

### BRIEF DESCRIPTION OF THE DRAWINGS

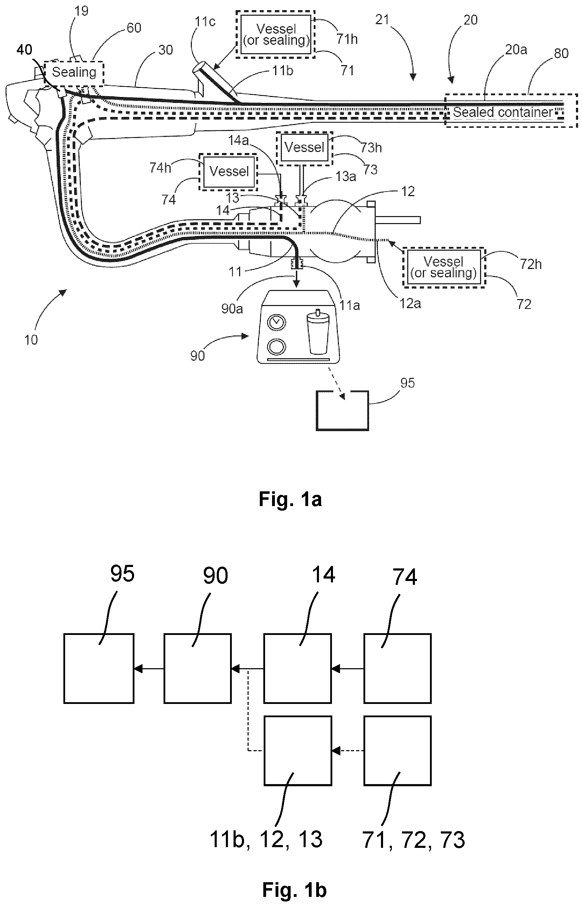

Preferred embodiments shall now be described with reference to the attached drawings, in which
- Fig. 1a:: shows a schematic depiction of a first exemplary embodiment of an arrangement for pre-cleaning an endoscope;
- Fig. 1 b:: shows a schematic flow diagram of a first exemplary embodiment of a method for pre-cleaning an endoscope;
- Fig. 2a:: shows a schematic depiction of a second exemplary embodiment of an arrangement for pre-cleaning an endoscope;
- Fig. 2b:: shows a schematic flow diagram of a second exemplary embodiment of a method for pre-cleaning an endoscope;
- Fig. 3a:: shows a schematic depiction of a third exemplary embodiment of an arrangement for pre-cleaning an endoscope;
- Fig. 3b:: shows a schematic depiction of water channel and air channel in the embodiment of an arrangement for pre-cleaning an endoscope shown in Fig. 3a;
- Fig. 3c:: shows a schematic flow diagram of a third exemplary embodiment of a method for pre-cleaning an endoscope;
- Fig. 4:: shows a schematic flow diagram of an exemplary embodiment of a method for pre-cleaning an endoscope.

### DETAILED DESCRIPTION

In the figures, elements with the same or comparable functions are indicated with the same reference numerals.

Fig. 1a shows a schematic depiction of a first exemplary embodiment of an arrangement for pre-cleaning an endoscope 10. The endoscope 10 can be pre-cleaned immediately or shortly after use of the endoscope 10. The endoscope 10 comprises an endoscope body 30 and channels 11, 12, 13, 14 for transporting fluids, namely a suction channel 11, an air channel 12, a water channel 13, and an auxiliary water channel 14.

The suction channel 11 is adapted to draw liquids and/or objects, such as for example a tissue sample, from the distal end 20 of the endoscope 10 through the endoscope 10. The suction channel 11 extends from a suction channel connection port 11a to the distal end 20 of the endoscope 10. The distal end 20 is the end portion of the endoscope 10 that is located at the very front of an insert section 21 of the endoscope 20.

The air channel 12 is adapted to provide air to the distal end 20 of the endoscope 10. The air channel 12 extends from an air channel connection port 12a to the distal end 20 of the endoscope 10.

The water channel 13 is adapted to provide water at the distal end 20 of the endoscope 10. The water channel 13 extends from a water channel connection port 13a to the distal end 20 of the endoscope 10.

The auxiliary water channel 14 is adapted to provide water at the distal end 20 of the endoscope 10. The auxiliary water channel 14 extends from an auxiliary water channel connection port 14a to the distal end 20 of the endoscope 10.

The connection ports 11a, 11c, 12a, 13a, 14a are adapted for connection of fluid hoses and/or vessels and/or closure elements. Each of the channels 11, 12, 13, 14 extends from one of the connection ports 11a, 12a, 13a, 14a to the distal end 20 of the endoscope 10.

The channels 11, 12, 13, 14 are fluidly connected to each other at the distal end 20 of the endoscope 10 by arranging a container 80 onto the distal end 20 of the endoscope 10. The container 80 is arranged onto the distal end 20 of the endoscope 10 in such a manner that the distal end 20, including the outer circumferential surface 20a of the distal end 20, is arranged entirely inside the container 80. If the container 80 gets filled with liquid, the whole distal end 20, including the outer surface 20a of the distal end 20, is in contact with the liquid so that a reliable pre-cleaning of the whole distal end 20 can be achieved. The container 80 is arranged on the distal end 20 of the endoscope 10 in such a manner that the distal end 20 of the endoscope 10 is sealed liquid-tight and gas-tight from the environment that surrounds the container 80.

The endoscope 20 furthermore comprises a biopsy branch 11b that extends from a biopsy branch connection port 11c to the suction channel 11.

The air channel 12 and the water channel 13 can be fluidly connected and/or separated from each other via an air/water cylinder 19. A vent can be assembled to the air/water cylinder 19. The air/water cylinder 19 is sealed with a closure element 60 that is adapted to close the air/water cylinder 19 fluid-tight. Furthermore, a suction cylinder 40 is provided to which the suction channel 11 is connected to. A vent can be assembled to the suction cylinder 40.

A vessel 71 is arranged onto the biopsy branch connection port 11c. If the biopsy branch 11b does not need to be flushed during the pre-cleaning procedure, a closure element can be arranged onto the biopsy branch connection port 11c instead of the vessel 71.

A vessel 72 is arranged onto the connection port 12a of the air channel 12. If the air channel 12 does not need to be flushed during the pre-cleaning procedure, a closure element can be arranged onto the connection port 12a instead of the vessel 72.

A vessel 73 is arranged onto the connection port 13a of the water channel 13. If the water channel 13 does not need to be flushed during the pre-cleaning procedure, a closure element can be arranged onto the connection port 13a instead of the vessel 73.

Furthermore, a vessel 74 is arranged onto the connection port 14a of the auxiliary water channel 14. If auxiliary water channel 14 does not need to be flushed during the pre-cleaning procedure, a closure element can be arranged onto the connection port 14a instead of the vessel 74.

The vessels 71, 72, 73, 74 are filled with liquid, in particular with a disinfectant solution and/or detergent solution.

A suction device 90 that is a suction pump in this preferred embodiment is connected via a fluid hose 90a to the connection port 11a of the suction channel 11. When the suction device 90 is activated, an underpressure is applied to the system so that liquid is drawn towards the suction device 90 as described in detail below.

The vessel 71 comprises a vessel holding chamber 71h with a vessel holding chamber volume for holding the liquid, wherein the vessel holding chamber volume reduces, preferably by the volume of liquid that is drawn from the vessel 71, during the drawing of liquid from the vessel 71 to the channel 11b. The vessel holding chamber 71h is completely filled with liquid at any time before all of the liquid has been drawn out of the vessel holding chamber 71h. The volume of the liquid in the vessel holding chamber 71h is reduced, but the vessel holding chamber 71h is still completely filled with liquid, because the volume of the liquid and the volume of the vessel holding chamber reduce in the same way.

The vessel 72 comprises a vessel holding chamber 72h with a vessel holding chamber volume for holding the liquid, wherein the vessel holding chamber volume reduces, preferably by the volume of liquid that is drawn from the vessel 72, during the drawing of liquid from the vessel 72 to the channel 12a. The vessel holding chamber 72h is completely filled with liquid at any time before all of the liquid has been drawn out of the vessel holding chamber 72h. The volume of the liquid in the vessel holding chamber 72h is reduced, but the vessel holding chamber 72h is still completely filled with liquid, because the volume of the liquid and the volume of the vessel holding chamber reduce in the same way.

The vessel 73 comprises a vessel holding chamber 73h with a vessel holding chamber volume for holding the liquid, wherein the vessel holding chamber volume reduces, preferably by the volume of liquid that is drawn from the vessel 73, during the drawing of liquid from the vessel 73 to the channel 13. The vessel holding chamber 73h is completely filled with liquid at any time before all of the liquid has been drawn out of the vessel holding chamber 73h. The volume of the liquid in the vessel holding chamber 73h is reduced, but the vessel holding chamber 73h is still completely filled with liquid, because the volume of the liquid and the volume of the vessel holding chamber reduce in the same way.

The vessel 74 comprises a vessel holding chamber 74h with a vessel holding chamber volume for holding the liquid, wherein the vessel holding chamber volume reduces, preferably by the volume of liquid that is drawn from the vessel 74, during the drawing of liquid from the vessel 74 to the channel 14. The vessel holding chamber 74h is completely filled with liquid at any time before all of the liquid has been drawn out of the vessel holding chamber 74h. The volume of the liquid in the vessel holding chamber 74h is reduced, but the vessel holding chamber 74h is still completely filled with liquid, because the volume of the liquid and the volume of the vessel holding chamber reduce in the same way.

Liquid is drawn from the vessel 71 through the biopsy branch 11b and from there further through the suction channel 11, out of the connection port 11a, via the fluid hose 90a to the suction device 90 and from the suction device 90 to the suction jar 95.

Liquid is drawn from the vessel 72 through the air channel 12 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 73 through the water channel 13 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 74 through the auxiliary water channel 14 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

When the liquid is drawn out of the vessel holding chambers 71h, 72h, 73h, 74h, the connection between the suction device 90 and the connection port 11a to which the suction device 90 had been connected can be disconnected. If the underpressure is removed this way, the liquid stays in the channels due to capillary action in the channels. In this way, the wet status of the endoscope channels can be maintained for a long time, i.e. as long as necessary. Such a wet status is particularly beneficial because a drying of potential soiling can be avoided and, in case of a detergent solution (in particular in case of enzymatic detergents) used as liquid, additionally the contact time and/or immersion time can be extended to improve cleaning efficacy.

Fig. 1b shows a schematic flow diagram of a first exemplary embodiment of a method for pre-cleaning an endoscope. The method for pre-cleaning the endoscope 10 as described in connection with Fig. 1a comprises fluidly connecting the channels 11, 11b, 12, 13, 14 of the endoscope 10 to each other at the distal end 20 of the endoscope 10 by arranging a container 80 onto the distal end 20 of the endoscope 10 and connecting a suction device 90 to a channel 11, wherein the suction device 90 is further connected to a suction container, formed as a suction jar 95. Vessels 74, 71, 72, 73 that contain liquid in vessel holding chambers 71h, 72h, 73h, 74h are arranged onto the connection ports of the channels 11b, 12, 13, 14. By activating the suction device 90, liquid is drawn in the direction indicated by the arrows, namely from the vessels 74, 71, 72, 73 through the channels 11b, 12, 13, 14 (to which the vessels 74, 71, 72, 73 are connected to) towards the distal end 20 and from the distal end 20 via the container 80 into the suction channel 11 and through the suction channel 11 to the suction pump 90 to be collected in the suction jar 95. Then, the liquid is drawn out of the vessel holding chambers 71h, 72h, 73h, 74h, until the fluid flow stops because the vessel holding chambers are emptied and the volume of the vessel holding chambers is reduced to a minimum. Then, the underpressure is removed and the liquid stays in the channels due to capillary action in the channels.

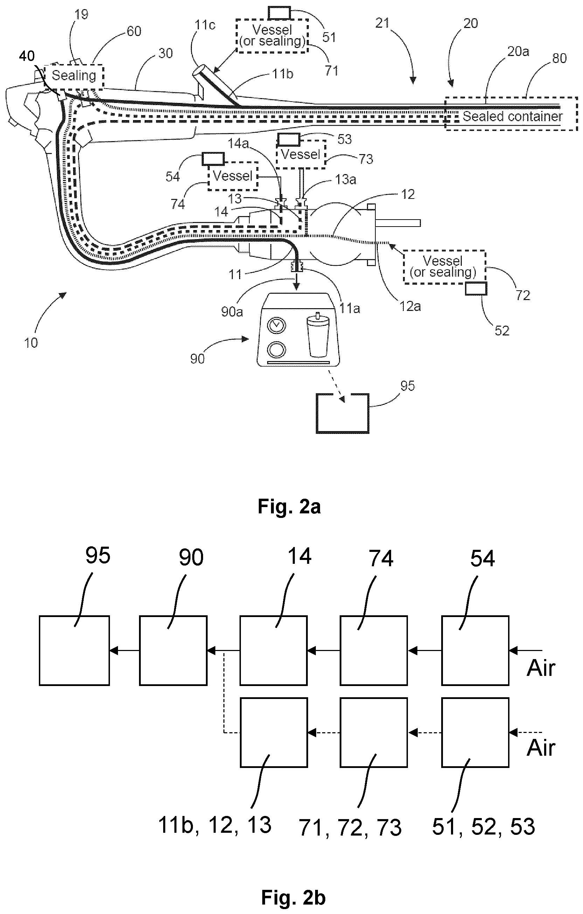

Fig. 2a shows a schematic depiction of a second exemplary embodiment of an arrangement for pre-cleaning an endoscope 10. The endoscope 10 can be pre-cleaned immediately or shortly after use of the endoscope 10. The endoscope 10 comprises an endoscope body 30 and channels 11, 12, 13, 14 for transporting fluids, namely a suction channel 11, an air channel 12, a water channel 13, and an auxiliary water channel 14.

The suction channel 11 is adapted to draw liquids and/or objects, such as for example a tissue sample, from the distal end 20 of the endoscope 10 through the endoscope 10. The suction channel 11 extends from a suction channel connection port 11a to the distal end 20 of the endoscope 10. The distal end 20 is the end portion of the endoscope 10 that is located at the very front of an insert section 21 of the endoscope 20.

The air channel 12 is adapted to provide air to the distal end 20 of the endoscope 10. The air channel 12 extends from an air channel connection port 12a to the distal end 20 of the endoscope 10.

The water channel 13 is adapted to provide water at the distal end 20 of the endoscope 10. The water channel 13 extends from a water channel connection port 13a to the distal end 20 of the endoscope 10.

The auxiliary water channel 14 is adapted to provide water at the distal end 20 of the endoscope 10. The auxiliary water channel 14 extends from an auxiliary water channel connection port 14a to the distal end 20 of the endoscope 10.

The connection ports 11a, 11c, 12a, 13a, 14a are adapted for connection of fluid hoses and/or vessels and/or closure elements. Each of the channels 11, 12, 13, 14 extends from one of the connection ports 11a, 12a, 13a, 14a to the distal end 20 of the endoscope 10.

The channels 11, 12, 13, 14 are fluidly connected to each other at the distal end 20 of the endoscope 10 by arranging a container 80 onto the distal end 20 of the endoscope 10. The container 80 is arranged onto the distal end 20 of the endoscope 10 in such a manner that the distal end 20, including the outer circumferential surface 20a of the distal end 20, is arranged entirely inside the container 80. If the container 80 gets filled with liquid, the whole distal end 20, including the outer surface 20a of the distal end 20, is in contact with the liquid so that a reliable pre-cleaning of the whole distal end 20 can be achieved. The container 80 is arranged on the distal end 20 of the endoscope 10 in such a manner that the distal end 20 of the endoscope 10 is sealed liquid-tight and gas-tight from the environment that surrounds the container 80.

The endoscope 20 furthermore comprises a biopsy branch 11b that extends from a biopsy branch connection port 11c to the suction channel 11.

The air channel 12 and the water channel 13 can be fluidly connected and/or separated from each other via an air/water cylinder 19. A vent can be assembled to the air/water cylinder 19. The air/water cylinder 19 is sealed with a closure element 60 that is adapted to close the air/water cylinder 19 fluid-tight. Furthermore, a suction cylinder 40 is provided to which the suction channel 11 is connected to. A vent can be assembled to the suction cylinder 40.

A vessel 71 is arranged onto the biopsy branch connection port 11c. If the biopsy branch 11b does not need to be flushed during the pre-cleaning procedure, a closure element can be arranged onto the biopsy branch connection port 11c instead of the vessel 71.

A vessel 72 is arranged onto the connection port 12a of the air channel 12. If the air channel 12 does not need to be flushed during the pre-cleaning procedure, a closure element can be arranged onto the connection port 12a instead of the vessel 72.

A vessel 73 is arranged onto the connection port 13a of the water channel 13. If the water channel 13 does not need to be flushed during the pre-cleaning procedure, a closure element can be arranged onto the connection port 13a instead of the vessel 73.

Furthermore, a vessel 74 is arranged onto the connection port 14a of the auxiliary water channel 14. If auxiliary water channel 14 does not need to be flushed during the pre-cleaning procedure, a closure element can be arranged onto the connection port 14a instead of the vessel 74.

The vessels 71, 72, 73, 74 are filled with liquid, in particular with a disinfectant solution and/or detergent solution.

A suction device 90 that is a suction pump in this preferred embodiment is connected via a fluid hose 90a to the connection port 11a of the suction channel 11. When the suction device 90 is activated, an underpressure is applied to the system so that liquid is drawn towards the suction device 90 as described in detail below.

Liquid is drawn from the vessel 71 through the biopsy branch 11b and from there further through the suction channel 11, out of the connection port 11a, via the fluid hose 90a to the suction device 90 and from the suction device 90 to the suction jar 95.

Liquid is drawn from the vessel 72 through the air channel 12 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 73 through the water channel 13 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 74 through the auxiliary water channel 14 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

The vessels 71, 72, 73, 74 do not necessarily have vessel holding chambers that can change their volumes. Instead the vessels of the arrangement shown in Fig. 2a can either have a constant, non-changing volume or a volume that can vary and change with the amount of liquid in the vessel. Furthermore, the vessels 71, 72, 73, 74 in the arrangement shown in Fig. 2a are not air-tight. Thus, air from the environment surrounding the vessels 71, 72, 73, 74 can enter inside the vessels and be transferred from the vessels through the channels towards the suction device. Therefore, hydrophobic filters 51, 52, 53, 54 are provided. A hydrophobic filter 51 is connected to the vessel 71. A hydrophobic filter 52 is connected to the vessel 72. A hydrophobic filter 53 is connected to the vessel 73. And a hydrophobic filter 54 is connected to the vessel 74. The hydrophobic filters 51, 52, 53, 54 are designed to allow gas, in particular air, to flow from the environment that surrounds the vessels 71, 72, 73, 74 via the hydrophobic filters 51, 52, 53, 54 into the vessel holding chambers of the corresponding vessels. The hydrophobic filters 51, 52, 53, 54 are designed to prevent liquid to flow from the environment that surrounds the vessels 71, 72, 73, 74 via the hydrophobic filters 51, 52, 53, 54 into the vessel holding chambers of the corresponding vessels 71, 72, 73, 74. With these hydrophobic filters 51, 52, 53, 54 at the vessels 71, 72, 73, 74 it is possible to draw liquid from the vessels 71, 72, 73, 74 through the channels and after the liquid has been drawn out of the vessels to automatically draw air through the channels, if the underpressure applied to draw the liquid through the channels is upheld.

Fig. 2b shows a schematic flow diagram of a second exemplary embodiment of a method for pre-cleaning an endoscope. The method for pre-cleaning the endoscope 10 as described in connection with Fig. 2a comprises fluidly connecting the channels 11, 11b, 12, 13, 14 of the endoscope 10 to each other at the distal end 20 of the endoscope 10 by arranging a container 80 onto the distal end 20 of the endoscope 10 and connecting a suction device 90 to a channel 11, wherein the suction device 90 is further connected to a suction container, formed as a suction jar 95. Vessels 74, 71, 72, 73 that contain liquid in vessel holding chambers are arranged onto the connection ports of the channels 11b, 12, 13, 14. By activating the suction device 90, liquid is drawn in the direction indicated by the arrows, namely from the vessels 74, 71, 72, 73 through the channels 11b, 12, 13, 14 (to which the vessels 74, 71, 72, 73 are connected to) towards the distal end 20 and from the distal end 20 via the container 80 into the suction channel 11 and through the suction channel 11 to the suction pump 90 to be collected in the suction jar 95. Then, the liquid is drawn out of the vessel holding chambers of the vessels until the no liquid is left in the vessels. Then, through the hydrophobic filters 51, 52, 53, 54 air enters the vessels and is drawn into and through the channels towards the suction device.

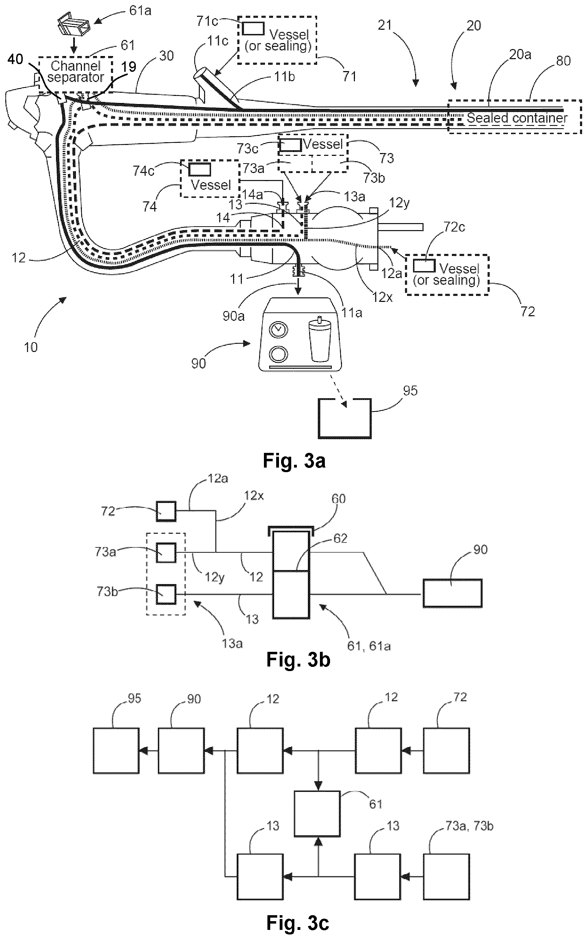

Fig. 3a shows a schematic depiction of a third exemplary embodiment of an arrangement for pre-cleaning an endoscope 10. The endoscope 10 can be pre-cleaned immediately or shortly after use of the endoscope 10. The endoscope 10 comprises an endoscope body 30 and channels 11, 12, 13, 14 for transporting fluids, namely a suction channel 11, an air channel 12, a water channel 13, and an auxiliary water channel 14.

The suction channel 11 is adapted to draw liquids and/or objects, such as for example a tissue sample, from the distal end 20 of the endoscope 10 through the endoscope 10. The suction channel 11 extends from a suction channel connection port 11a to the distal end 20 of the endoscope 10. The distal end 20 is the end portion of the endoscope 10 that is located at the very front of an insert section 21 of the endoscope 20.

The air channel 12 is adapted to provide air to the distal end 20 of the endoscope 10. The air channel 12 extends from an air channel connection port 12a to the distal end 20 of the endoscope 10.

The water channel 13 is adapted to provide water at the distal end 20 of the endoscope 10. The water channel 13 extends from a water channel connection port 13a to the distal end 20 of the endoscope 10.

The auxiliary water channel 14 is adapted to provide water at the distal end 20 of the endoscope 10. The auxiliary water channel 14 extends from an auxiliary water channel connection port 14a to the distal end 20 of the endoscope 10.

The connection ports 11a, 11c, 12a, 13a, 14a are adapted for connection of fluid hoses and/or vessels and/or closure elements. Each of the channels 11, 12, 13, 14 extends from one of the connection ports 11a, 12a, 13a, 14a to the distal end 20 of the endoscope 10.

The channels 11, 12, 13, 14 are fluidly connected to each other at the distal end 20 of the endoscope 10 by arranging a container 80 onto the distal end 20 of the endoscope 10. The container 80 is arranged onto the distal end 20 of the endoscope 10 in such a manner that the distal end 20, including the outer circumferential surface 20a of the distal end 20, is arranged entirely inside the container 80. If the container 80 gets filled with liquid, the whole distal end 20, including the outer surface 20a of the distal end 20, is in contact with the liquid so that a reliable pre-cleaning of the whole distal end 20 can be achieved. The container 80 is arranged on the distal end 20 of the endoscope 10 in such a manner that the distal end 20 of the endoscope 10 is sealed liquid-tight and gas-tight from the environment that surrounds the container 80.

The endoscope 20 furthermore comprises a biopsy branch 11b that extends from a biopsy branch connection port 11c to the suction channel 11.

The air channel 12 and the water channel 13 can be fluidly connected and/or separated from each other via an air/water cylinder 19. A vent can be assembled to the air/water cylinder 19. Furthermore, a suction cylinder 40 is provided to which the suction channel 11 is connected to. A vent can be assembled to the suction cylinder 40.A vessel 71 is arranged onto the biopsy branch connection port 11c. If the biopsy branch 11b does not need to be flushed during the pre-cleaning procedure, a closure element can be arranged onto the biopsy branch connection port 11c instead of the vessel 71.

A vessel 72 is arranged onto the connection port 12a of the air channel 12. If the air channel 12 does not need to be flushed during the pre-cleaning procedure, a closure element can be arranged onto the connection port 12a instead of the vessel 72.

A vessel 73 is arranged onto the connection port 13a of the water channel 13. If the water channel 13 does not need to be flushed during the pre-cleaning procedure, a closure element can be arranged onto the connection port 13a instead of the vessel 73.

Furthermore, a vessel 74 is arranged onto the connection port 14a of the auxiliary water channel 14. If auxiliary water channel 14 does not need to be flushed during the pre-cleaning procedure, a closure element can be arranged onto the connection port 14a instead of the vessel 74.

The vessels 71, 72, 73, 74 are filled with liquid, in particular with a disinfectant solution and/or detergent solution.

A suction device 90 that is a suction pump in this preferred embodiment is connected via a fluid hose 90a to the connection port 11a of the suction channel 11. When the suction device 90 is activated, an underpressure is applied to the system so that liquid is drawn towards the suction device 90 as described in detail below.

Liquid is drawn from the vessel 71 through the biopsy branch 11b and from there further through the suction channel 11, out of the connection port 11a, via the fluid hose 90a to the suction device 90 and from the suction device 90 to the suction jar 95.

Liquid is drawn from the vessel 72 through the air channel 12 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 73 through the water channel 13 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 74 through the auxiliary water channel 14 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

In this embodiment, the vessel 73 has a water part 73a and an air part 73b. The water part 73a is connected to the part of the connection port 13a that is connected to the water channel 13. The air part 73b is connected to the part of the connection port 13a that is connected to the air channel branch 12y of the air channel 12. The other air channel branch 12x is connected to the vessel 72 via the connection port 12a. These two air channel branches 12x, 12y join together and then form the air channel 12.

Because the water channel 13 and the air channel 12 are connected to each other via the air/water cylinder 19, a channel separator 61a can be connected to the air/water cylinder 19. This channel separator 61a is adapted to provide a channel separation 61, i.e. to separate the water channel 13 from the air channel 12. This channel separator 61a serves as a channel separator 61 by providing a sealing element 62 (see Fig. 3b) between the water channel 13 and the air channel 12 so that a fluid connection between the air channel 12 and the water channel 13 is disconnected. The channel separator 61a also comprises a closure element 60 (see Fig. 3b) to provide a sealing between the channels 12, 13 and the environment surrounding the endoscope in the area of the air/water cylinder 19.

Each of the vessels 71, 72, 73, 74 contains a code 71c, 72c, 73c, 74c, wherein the codes are only readable if no liquid is left in the corresponding vessel and not readable if liquid is left in the corresponding vessel. With such codes it can be determined and documented whether the liquid has been drawn out of the vessels through the channels and thus, it can be determined and documented whether the channels have been flushed with the liquid.

Fig. 3b shows a schematic depiction of water channel 13 and air channel 12 in the embodiment of an arrangement shown in Fig. 3a. As described in connection with Fig. 3a, the channel separator 61a forms a channel separation 61 that has a sealing element 62 that separates the air channel 12 from the water channel 13. Furthermore, a closure element 60 is provided to seal the channels from the air of the environment.

The suction device 90 is connected to the channel system, preferably to the suction channel 13, wherein the channels are fluidly connected to each other via a container 80 arranged at the distal end 20 of the endoscope 10 (as shown in Fig. 3a). However, the suction device 90 can alternatively be connected to the distal end 20 of the endoscope 10 directly or via a hose or the like that is connected to the distal end 20 of the endoscope 10.

After a defined runtime of the suction device 90, the vessels 72, 73a, 73b are emptied, i.e. the liquid is drawn out of the vessels 72, 73a, 73b through the channels 12x, 12y, 12, 13, if no blockage is present in the channels 12x, 12y, 12, 13 that prevents liquid flow through the channels 12x, 12y, 12, 13. The user can verify whether the vessels are empty, i.e. whether the liquid has been drawn out of each of the vessels and thus check whether one of these channels is blocked and in case at least one of the channels is blocked, determine which channel or which channels are blocked. The vessels are preferably transparent so that it can easily be seen whether and how much liquid is left in the vessels.

If, after having applied an underpressure to flush the channels 12x, 12y, 12, 13, liquid remains in the vessel 72 and no liquid is left in the vessel 73a and no liquid is left in the vessel 73b, the first branch of the air channel 12x to which the vessel 72 is connected is blocked.

If, after having applied an underpressure to flush the channels 12x, 12y, 12, 13, liquid remains in the vessel 73a and no liquid is left in the vessel 72 and no liquid is left in the vessel 73b, the second branch of the air channel 12y to which the vessel 73a is connected is blocked.

If, after having applied an underpressure to flush the channels 12x, 12y, 12, 13, liquid remains in the vessel 72 and liquid remains in the vessel 73a and no liquid is left in the vessel 73b, either the first branch 12x and the second branch 12y of the air channel or the air channel 12 is blocked.

If, after having applied an underpressure to flush the channels, liquid remains in the vessel 73b and no liquid is left in the vessel 72 and no liquid is left in the vessel 73a, the water channel 13 to which the vessel 73b is connected is blocked.

If, after having applied an underpressure to flush the channels 12x, 12y, 12, 13, liquid remains in the vessel 72 and liquid remains in the vessel 73a and liquid remains in the vessel 73b, the fluid connection that is provided at the distal end 20 is blocked.

Fig. 3c shows a schematic flow diagram of an exemplary embodiment of a method for pre-cleaning an endoscope, wherein an arrangement as described in Fig. 3a and Fig. 3b is provided. The vessels 72, 73a, 73b are transparent and/or contain a code that can only be read out if the vessels are empty. Due to an underpressure applied by the suction pump 90, liquid is drawn out of the vessels 72, 73a, 73b through the air channel 12 and through the water channel 13. As described in connection with Fig. 3a and Fig. 3b, a channel separator 61 separates the water channel 13 from the air channel 12 so that these channels are disconnected. The liquid is then drawn through these channels 12, 13 towards the suction pump 90 and collected in a suction jar 95.

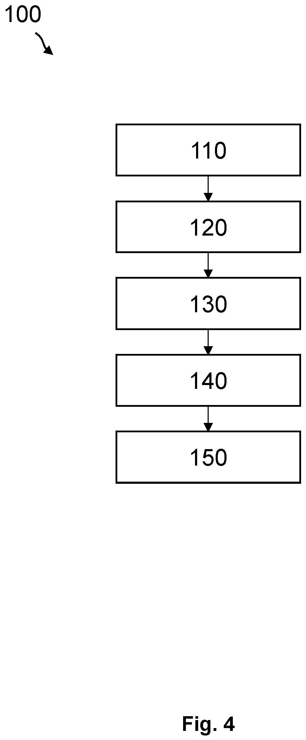

Fig. 4 shows a schematic flow diagram of an exemplary embodiment of a method for pre-cleaning an endoscope as shown in Fig. 1a or as shown in Fig. 2a or as shown in Fig. 3a. The method 100 is conducted immediately or shortly after use of the endoscope. The method comprises the following steps:
In a step 110, providing an endoscope that comprises channels for transporting fluids, connection ports, in particular for connection of fluid hoses, and a distal end of the endoscope, wherein each of the channels extends from one of the connection ports to the distal end of the endoscope.

In a step 120, fluidly connecting the channels to each other at the distal end of the endoscope.

In a step 130, connecting a suction device, preferably a suction pump, to at least one of the connection ports or to the distal end of the endoscope.

In a step 140, connecting at least one vessel that holds liquid to at least one of the connection ports.

In a step 150, flushing at least one of the channels with liquid by activating the suction device so that liquid is drawn from the at least one vessel through the at least one channel towards the suction device.

## Claims

1. A method for pre-cleaning an endoscope, preferably immediately or shortly after use of the endoscope, the method comprising:
- providing an endoscope that comprises
∘ channels for transporting fluids,
∘ connection ports, in particular for connection of fluid hoses,
∘ and a distal end of the endoscope,
wherein each of the channels extends from one of the connection ports to the distal end of the endoscope;
- fluidly connecting the channels to each other at the distal end of the endoscope;
- connecting a suction device, preferably a suction pump, to at least one of the connection ports or to the distal end of the endoscope;
- connecting at least one vessel that holds liquid to at least one of the connection ports;
- flushing at least one of the channels with liquid by activating the suction device so that liquid is drawn from the at least one vessel through the at least one channel towards the suction device.

2. The method according to the preceding claim,
wherein the at least one vessel is fluidly connected to at least one of the connection ports in such a manner that the at least one vessel is sealed liquid-tight from the environment that surrounds the vessel, and/or wherein the at least one vessel is fluidly connected to at least one of the connection ports in such a manner that the at least one vessel is sealed gas-tight from the environment that surrounds the vessel.

3. The method according to at least one of the preceding claims,
wherein the at least one vessel comprises a vessel holding chamber with a vessel holding chamber volume for holding the liquid,
wherein the vessel holding chamber volume reduces, preferably by the volume of liquid that is drawn from the vessel, during the drawing of liquid from the vessel to the at least one channel,
wherein preferably the vessel holding chamber is completely filled with liquid at any time before all of the liquid has been drawn out of the vessel holding chamber.

4. The method according to at least one of the preceding claims,
wherein the liquid is drawn out of the vessel holding chamber until the vessel holding chamber volume reaches its minimal volume so that remaining liquid in the at least one channel stops to flow towards the suction device, and/or wherein the vessel holding chamber is formed by a plastic bag, and/or wherein the vessel comprises a height-adjustable structure that reduces its height while liquid is drawn out of the vessel holding chamber.

5. The method according to at least one of the preceding claims, comprising:
- when the liquid is drawn out of the vessel holding chamber, disconnecting the connection between the suction device and the at least one connection port to which the suction device had been connected.

6. The method according to at least one of the preceding claims,
wherein a hydrophobic filter is arranged at each of the at least one vessel, wherein the hydrophobic filter is designed to allow gas to flow from the environment that surrounds the vessel via the hydrophobic filter into the vessel holding chamber of the corresponding vessel.

7. The method according to the preceding claim, comprising
- after flushing at least one of the channels with liquid, flushing at least one of the channels with air by keeping the suction device activated after the liquid has been drawn out from the at least one vessel to draw air from the environment that surrounds the vessel via the hydrophobic filter into the vessel holding chamber of the corresponding vessel and further through the at least one channel towards the suction device.

8. The method according to at least one of the preceding claims, comprising:
wherein fluidly connecting the channels to each other at the distal end of the endoscope is conducted by arranging a container onto the distal end of the endoscope, and/or
wherein when flushing at least one of the channels with a liquid, liquid is drawn from the at least one vessel through the at least one channel via the distal end towards the suction device.

9. The method according to at least one of the preceding claims,
wherein the channels comprise
- an air channel that extends from an air channel connection port to the distal end of the endoscope,
- a suction channel that extends from a suction channel connection port to the distal end of the endoscope,
- a water channel that extends from a water channel connection port to the distal end of the endoscope,
- and preferably an auxiliary water channel that extends from an auxiliary water channel connection port to the distal end of the endoscope.

10. The method according to at least one of the preceding claims, comprising:
- disconnecting a fluid connection between the air channel and the water channel, preferably by arranging a sealing element at an air/water cylinder, for separating the air channel from the water channel;
- and preferably drawing liquid from the at least one vessel through at least one of the channels towards the suction device.

11. The method according to the preceding claim, comprising:
- checking, preferably after a predetermined time of activation of the suction device, whether liquid is left in each of the vessels, wherein preferably each of the vessels contains a code that is readable if no liquid is left in the vessel, and preferably not readable if liquid is left in the vessel, wherein preferably the patency of the channel that is connected to the vessel is determined and/or proofed by reading out the code.

12. The method according to at least one of the preceding claims, comprising:
- assessing, preferably after a predetermined time of activation of the suction device, whether liquid is left in each of the vessels,
- and dependent on the result of the assessment, determining whether at least one of the channels is blocked,
- and preferably if at least one of the channels is blocked, determining which of the channels is blocked.

13. The method according to at least one of the preceding claims,
wherein fluidly connecting the channels to each other at the distal end of the endoscope is conducted by arranging a container onto the distal end of the endoscope,
wherein the container is arranged on the distal end of the endoscope in such a manner that the distal end of the endoscope is sealed liquid-tight, and preferably gas-tight, from the environment that surrounds the container, wherein preferably each of the connection ports is connected to one of the at least one vessel except for the connection port that is connected to the suction device.

14. An arrangement for pre-cleaning an endoscope, preferably immediately or shortly after use of the endoscope, in particular for pre-cleaning an endoscope according to the method according to at least one of the preceding claims, the arrangement comprising:
- an endoscope that comprises
∘ channels for transporting fluids,
∘ connection ports, in particular for connection of fluid hoses,
∘ and a distal end of the endoscope,
wherein each of the channels extends from one of the connection ports to the distal end of the endoscope;
- at least one vessel that holds liquid, wherein the at least one vessel is connected to at least one of the connection ports;
- a suction device, preferably a suction pump, that is connected to one of the connection ports or to the distal end of the endoscope.

15. The arrangement according to at least one of the preceding claims, comprising:
- a container that is arranged onto the distal end of the endoscope in such a manner that the channels are fluidly connected to each other at the distal end of the endoscope,
- wherein the container is arranged on the distal end of the endoscope in such a manner that the distal end of the endoscope is sealed liquid-tight, and preferably gas-tight, from the environment that surrounds the container,
- wherein preferably each of the connection ports is connected to one of the at least one vessel except for the connection port that is connected to the suction device.
